# EUROPEAN PATENT SPECIFICATION

(11) **EP 2 510 907 B1**
(45) Date of publication and mention of the grant of the patent: **27.08.2014**
(21) Application number: 12163278.0
(22) Date of filing: 05.04.2012
(51) Int. Cl.: A61F 2/90, A61F 2/06

(54) **Stent**
Stent
Endoprothèse

(30) Priority: 14.04.2011 JP 2011090495
(43) Date of publication of application: 17.10.2012
(73) Proprietor: ASAHI INTECC CO., LTD., Nagoya-shi, Aichi 463-0024 (JP)
(72) Inventor: Nishigishi, Makoto, Nagoya-shi, Aichi 463-0024 (JP)
(74) Representative: Winter, Brandl, Fürniss, Hübner, Röss, Kaiser, Polte - Partnerschaft mbB

(56) References cited:
- WO-A1-2010/120926
- GB-A- 2 470 484
- US-A1- 2002 179 166

## Description

### Technical Field

The present invention relates to a stent.

### Background Art

In the related art, there is known a stent as a medical instrument used to restore or suppress the bloodstream. The stent is indwelt in, for example, a stenosis in the vessel. The indwelt stent restores the bloodstream by keeping the inner diameter of the stenosis in the vessel constant. Alternatively, the stent is indwelt at an inlet of an aneurysm. The indwelt stent suppresses the bloodstream flowing into the aneurysm.

Such a stent is disclosed in, for example, JP-A-11-57021 US6719934B2 or JP-A-2007-518520. The stents disclosed in said patent documents are each in the shape of a cylindrical basket. That is, each stent is formed by weaving a plurality of strands together.

The diameter of the stent disclosed in JP-A-11-57021 can easily be set to an arbitrary value. Therefore, the stent can easily be indwelt in a lesion site.

However, the stent disclosed in the above patent document has the following matter to be considered. That is, the stent indwelt at a lesion site is hardly effective for stabilizing the bloodstream inside an inner cavity of the stent (hereinafter also referred to as stent inner-cavity bloodstream). For example, the stent indwelt at an inlet of an aneurysm cannot fully suppress the bloodstream flowing into the aneurysm.

US 2002/0179166 A1 discloses a stent which, in accordance with the preamble of claim 1, comprises a first strand group and a second strand group which are woven together, wherein a plurality of second strands forming the second strand group are each larger in diameter than a plurality of first strands forming the first strand group.

### Summary of Invention

The present invention has the object to provide a stent which is excellent in stabilizing a stent inner-cavity bloodstream. This object is solved by a stent according to claim 1.

A stent of the present invention includes a first strand group and a second strand group which are woven together,
wherein the first strand group is constituted by a plurality of first strands wound in a right-handed spiral around a virtual central axis that extends in a longitudinal direction of the stent,
the second strand group is constituted by a plurality of second strands wound in a left-handed spiral around the virtual central axis wherein the number of the second strands is larger than the number of the first strands, and
the plurality of second strands constituting the second strand group are each larger in diameter than each of the plurality of first strands constituting the first strand group.
Generally, the "diameter" corresponds to a maximum linear extension of the cross-sectional area of the respective one of the first and seconds strands, i.e., in analogy to the so-called Feret-diameter, to a longest distance between any two points on the contour of the cross-sectional area of the respective one of the first and seconds strands. Thus, where the respective one of the first and second strands has a circular cross-sectional area, the "diameter" corresponds to the diameter of the contour of the circular cross-sectional area of the respective one of the first and second strands, and where the respective one of the first and second strands has a non-circular cross-sectional area, the "diameter" corresponds to the diameter of a circle surrounding the contour of the non-circular cross-sectional area of the respective one of the first and second strands.
Further, among the plurality of first strands constituting the first strand group the diameter of one of the first strands may be different to or may be the same as the diameter of another one of the first strands, as long as each of the first strands has a smaller diameter than each of the second strands. Specifically, the first strands may each have a same (first) diameter, or the diameter may vary among the first strands. Similarly, among the plurality of second strands constituting the second strand group the diameter of one of the second strands may be different to or may be the same as the diameter of another one of the second strands, as long as each of the second strands has a larger diameter than each of the first strands. Specifically, the second strands may each have a same (second) diameter, or the diameter may vary among the second strands.

The stent of the present invention further includes a densely woven part having a larger total number of the first strands and second strands; and
a sparsely woven part having a smaller total number of the first strands and second strands,
wherein in the sparsely woven part, the number of the first strands is less than the number of the second strands.

Further, in the stent of the present invention, the densely woven part is formed at each of the ends of the stent in a longitudinal direction thereof, and the sparsely woven part is formed at a central part of the stent in a longitudinal direction thereof.

### Brief Description of Drawings

The foregoing and other features, aspects and advantages of the invention will become more apparent from the following detailed description when taken in conjunction with the accompanying drawings.
Fig. 1 is a plan view schematically illustrating a stent according to an example not covered by the claims;
Fig. 2A is a cross-sectional view taken along line A-A of the stent shown in Fig. 1;
Fig. 2B is a cross-sectional view taken along line B-B of the stent shown in Fig. 1;
Fig. 3 is a schematic view illustrating an example of a method for using the stent illustrated in Fig. 1; and
Fig. 4 is a plan view schematically illustrating a stent according to an embodiment of the present invention.

### Description of Examples and Embodiment

Preferred examples and embodiment of the present invention are described below with reference to the accompanying drawings, in which like reference characters designate similar or identical parts throughout the several views thereof.

### (Example)

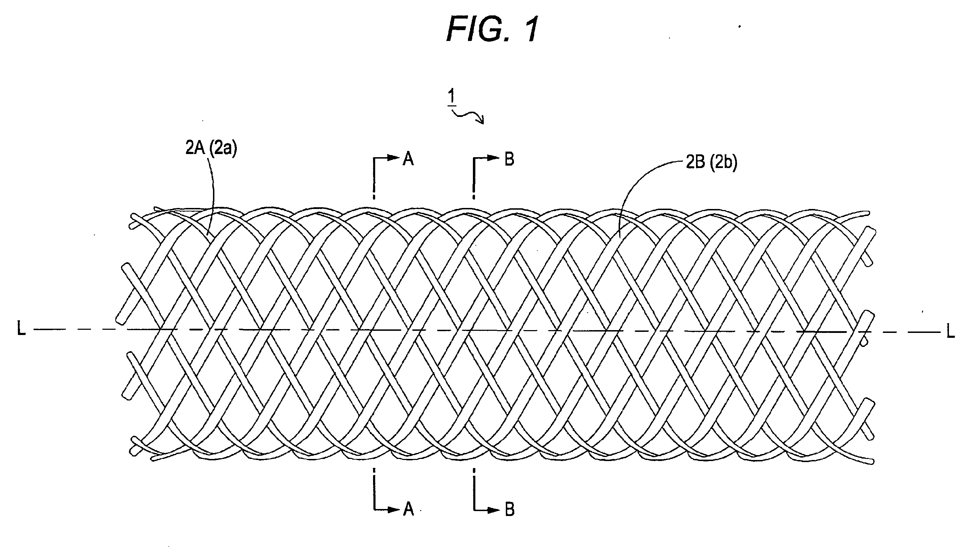

First, a stent according to an example not covered by the claims will be described below with reference to Figs. 1 to 3.
Fig. 1 is a plan view schematically illustrating a stent 1 according to the example.

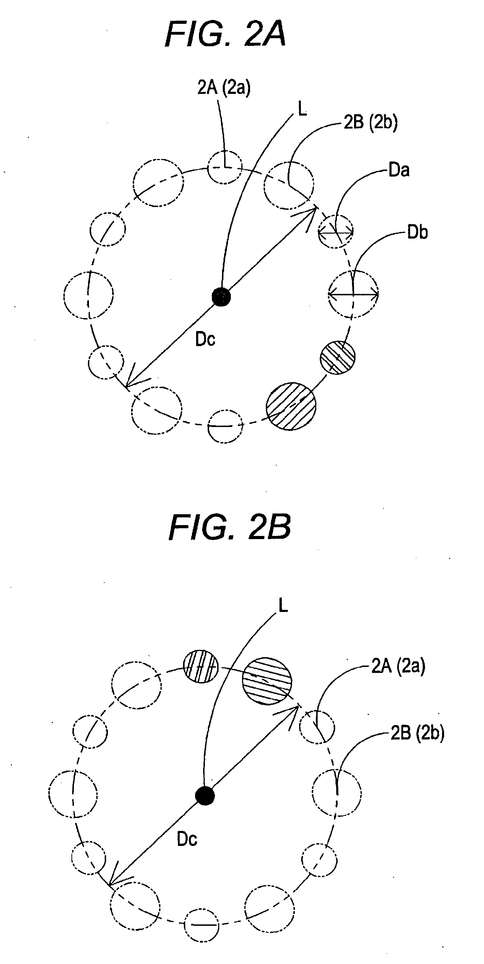

Fig. 2A is a cross-sectional view of the stent 1 taken along line A-A in Fig. 1, and Fig. 2B is a cross-sectional view of the stent 1 taken along line B-B in Fig. 1.

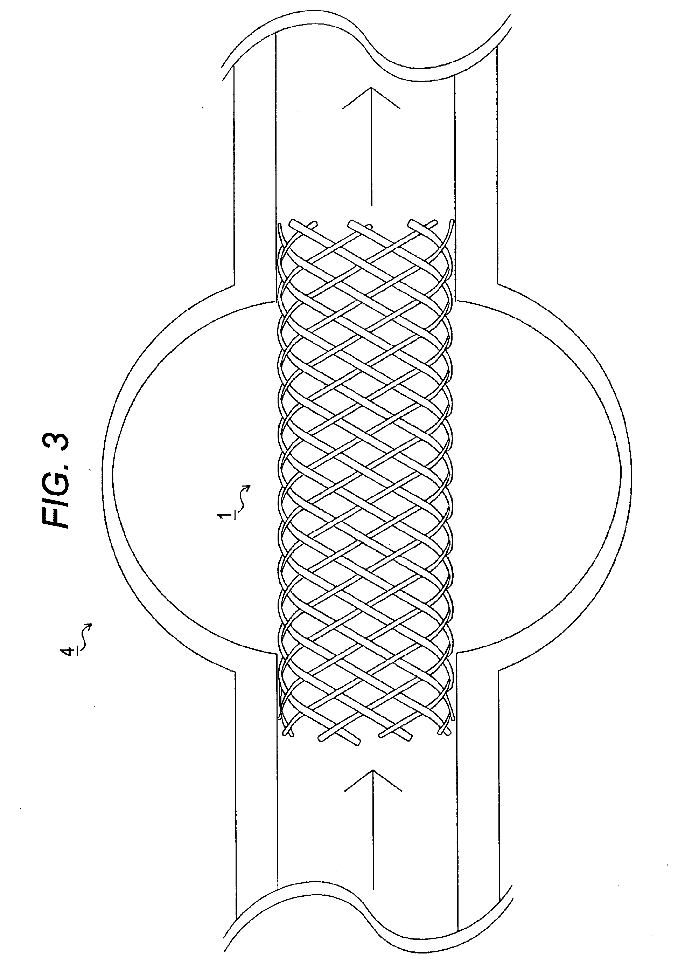

Fig. 3 is a schematic view illustrating an example of a method for using the stent 1 illustrated in Fig. 1.
In the example illustrated in Figs. 1 to 3, a first diameter Da of each one of a plurality of first strands 2a constituting a first strand group 2A is smaller than a second diameter Db of each one of a plurality of second strands 2b constituting a second strand group 2B. That is, the second diameter Db of each one of the plurality of second strands 2b constituting the second strand group 2B is larger than the first diameter Da of each one of the plurality of first strands 2a constituting the first strand group 2A. However, the configuration of the stent 1 is not limited to the above example as long as each of the diameters of the first strands constituting the first strand group is different from each of the diameters of the second strands constituting the second strand group. For example, the each of the first diameters of the first strands constituting the first strand group may be larger than each of the second diameters of the second strands constituting the second strand group.
In the description given below, the strand having the larger diameter is also referred to as a thick strand. On the other hand, the strand having the smaller diameter is also referred to as a thin strand.
In Figs. 2A and 2B, only one each of the first strand and the second strand is shaded for easy understanding of the relationship therebetween.

As illustrated in Fig. 1, the stent 1 includes a first strand group 2A and a second strand group 2B which are woven together.
More specifically, the first strand group 2A is wound in a right-handed spiral around a virtual central axis L that extends in the longitudinal direction of the stent 1. The second strand group 2B, on the other hand, is wound in a left-handed spiral around the virtual central axis L.
In this manner, in the stent 1 of the present embodiment, first strands 2a constituting the first strand group 2A wound in a right-handed spiral and second strands 2b constituting the second strand group 2B wound in a left-handed spiral are woven together. Therefore, the stent 1 of the present embodiment has a sufficient strength compared to a stent in the shape of a right- or left-handed coil where the strands are spirally wound only in one direction.

As illustrated in Figs. 2A and 2B, the diameter Da of each of the first strands 2a constituting the first strand group 2A is different from the diameter Db of each of the second strand 2b constituting the second strand group 2B.
More specifically, in the example, the diameter Da of each of the first strands 2a constituting the first strand group 2A is smaller than the diameter Db of each of the second strands 2b constituting the second strand group 2B. In other words, the diameter Db of each of the second strands 2b constituting the second strand group 2B is larger than the diameter Da of each of the first strands 2a constituting the first strand group 2A.
The stent diameter Dc is defined as the diameter of a circle obtained by drawing a virtual curved line passing through the central axis of each first strand 2a and the central axis of each second strand 2b adjacent to a first strand 2a, in the stent 1 to which no external force is applied (i.e., the stent in a free state). In the cut surface of the stent 1 having the stent diameter Dc, the circumference portion of each of the second strands 2b having the larger diameter protrudes toward the virtual center (virtual central axis L) more evidently than circumference portion of each of the first strands 2a.
That is, a left-handed spiral protrusion is formed of the circumference portion (i.e., outer peripheral portion) of each of the second strands 2b, protruding toward the virtual center (virtual central axis L) more than the circumference portion (i.e., outer peripheral portion) of each of the first strands 2a. On the other hand, a right-handed spiral protrusion is formed of the circumference portion (i.e., outer peripheral portion) of each of the first strands 2a protruding toward the virtual center (virtual central axis L) less than the circumference portion (i.e., outer peripheral portion) of each of the second strands 2b. In other words, the left-handed spiral protrusion has a larger radial height than the right-handed spiral protrusion toward the virtual center (virtual central axis L).
As illustrated in Fig. 3, therefore, in the stent 1 indwelt at a lesion site (aneurysm 4), the second strands 2b (left-handed spiral protrusions) more evidently protruding toward the virtual center (virtual central axis L) function as a left-handed spiral deflector. Therefore, the inner-cavity bloodstream of the stent 1 is turned into a left-handed spiral flow. As a result, the bloodstream is stabilized. Note that in Fig. 3, the bloodstream is shown by an arrow pointed in one direction.
With this configuration, the stent 1 indwelt at the inlet of the aneurysm 4 sufficiently suppresses the bloodstream flowing into the aneurysm 4.

As illustrated in Fig. 1, the first strands 2a constituting the first strand group 2A are each in the shape of a rod with a circular cross section.
For example, about several to several tens of the first strands 2a may be used.

The material for forming the first strands 2a may be, for example, stainless steel; super elastic alloys such as, a Ni-Ti alloy, a Cu-Al-Ni alloy, or a Cu-Zn-Al alloy; a piano wire; tungsten; or synthetic resins such as polyester, polyurethane, polyolefin, polytetrafluoroethylene, or a silicon resin.
Examples of the stainless steel include martensite-based stainless steel, ferrite-based stainless steel, austenite-based stainless steel, austenitic-ferritic duplex stainless steel, and precipitation-hardened stainless steel.

As illustrated in Fig. 1, the second strands 2b constituting the second strand group 2B are each in the shape of a rod with a circular cross section.
For example, about several to several tens of the second strands 2b may be used.

The material for the second strands 2b may be the same as or different from that for the first strand 2a.
In the case where the material for the first strands 2a is different from that for the second strands 2b, various characteristics of the materials for the respective strands can be exerted in combination.

The total number of the first strands 2a is equal to the total number of the second strands 2b.
Further, in the stent 1 of the example the plurality of first strands 2a have each a same (first) diameter Da, and the plurality of second strands 2b have each a same (second) diameter Db which is different from the first diameter Da. The left-handed spiral protrusions formed by the second strands 2b protruding toward the virtual center (virtual central axis L) have each a lager (radial) height than the right-handed spiral protrusions formed by the first strands 2a protruding toward the virtual center (virtual central axis L). Especially, each height of the left-handed protrusions and each height of the right-handed protrusions is regulated to substantially uniform size. Therefore, in the stent 1 of the example indwelt at a lesion site, the bloodstream is more stabilized by being turned into a left-handed spiral flow by the left-handed protrusions functioning as a left-handed spiral deflector having substantially uniform height.

The stent 1 of the example having the above configuration is formed of the first strand group 2A and the second strand group 2B which are woven together. Therefore, the strands constituting the respective strand groups are slidable relative to each other. That is, the diameter of the stent 1 can be set to an arbitrary value by sliding these strands. In other words, the diameter of the stent 1 can be increased or decreased, as necessary, by sliding these strands.
Therefore, in the case where the stent 1 is to be inserted into a tubular organ with an inner diameter smaller than the diameter Dc of the stent 1 in the free state, the diameter Dc of the stent 1 is decreased by sliding the strands before the stent 1 is inserted into the tubular organ.

The stent of the example may be used as follows, for example.
The diameter of the stent of the present embodiment is decreased. The stent with the decreased diameter is accommodated in a catheter. Then, the catheter is guided to and positioned on a lesion site along a guidewire.
After positioning the catheter, the stent is let out of the catheter and indwelt at the lesion site.
Note that the stent of the example may be guided to the lesion site using a balloon catheter.

The stent of the example may be manufactured as follows, for example. That is, a plurality of thin strands to constitute a first strand group and a plurality of thick strands to constitute a second strand group are prepared. Next, the plurality of thin strands is set in a stent weaving machine as right-handed spiral strands. Meanwhile, the plurality of thick strands is set in the stent weaving machine as left-handed spiral strands. After that, the stent weaving machine is operated to alternately weave the respective strands into the shape of a cylinder. As a result, a stent according to the example is obtained.

The functions and effects of the stent of the example will be described below.
(1) In the stent of the example, the first strands wound in a right-handed spiral and the second strands wound in a left-handed spiral are woven together. Therefore, the stent has a sufficient strength compared to the stent in the shape of a right- or left-handed coil where the strands are spirally wound only in one direction (i.e., either in the shape of a right-handed coil or a left-handed coil).
(2) A left-handed spiral protrusion formed of the second strand protruding toward the virtual central axis has a larger height than a right-handed spiral protrusion formed of the first strand protruding toward the virtual central axis.
   Therefore, in the stent indwelt at a lesion site, the left-handed spiral protrusion more evidently protruding toward the virtual central axis functions as a left-handed spiral deflector. As a result, the stent inner-cavity bloodstream is stabilized by being turned into a left-handed spiral flow.

### (Modification of the example)

A stent according to a modification of the example will be described below with reference to the drawing. The stent of the modification has the same configuration as the stent of the example except for the following features. That is, the stent of the modification has a densely woven pant and a sparsely woven part. The total number of the first and second strands in the densely woven part is larger than that in the sparsely woven part. Of the first strands and the second strands in the sparsely woven part, the number of the strands having each the smaller diameter is less than the number of the strands having each the larger diameter.
Therefore, the overlapping features with the stent of the example will not be described.

### (Embodiment)

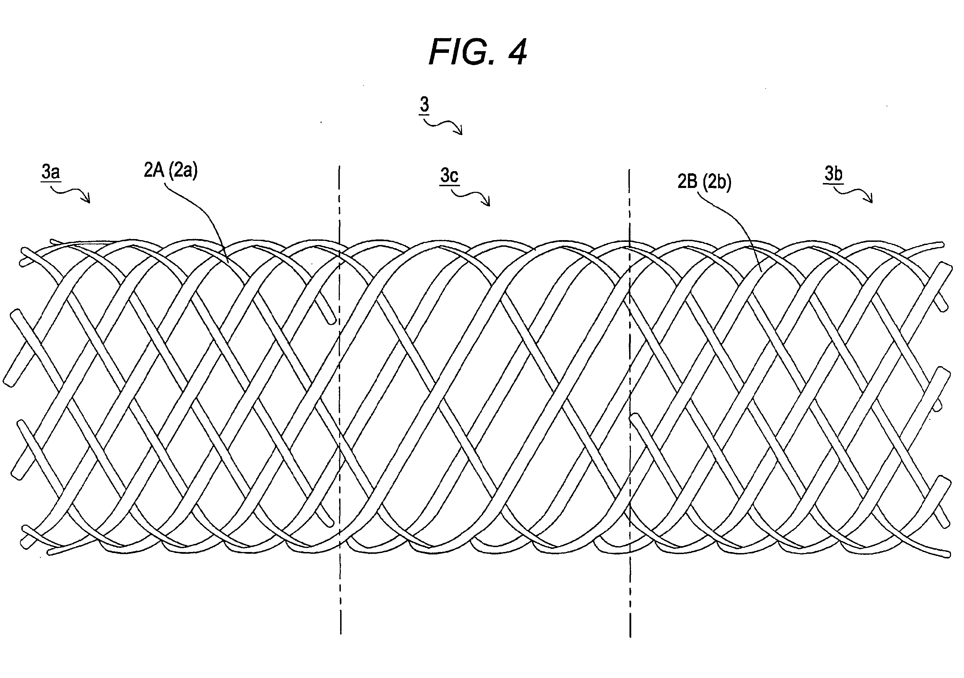

Fig. 4 is a plan view schematically illustrating the stent according to an embodiment of the present invention.

A stent 3 of the embodiment illustrated in Fig. 4 includes densely woven parts 3a and 3b formed at both ends of the stent 3 in a longitudinal direction thereof, and a sparsely woven part 3c formed in the middle of the stent 3 in a longitudinal direction thereof.

In the densely woven parts 3a and 3b, the total number of the first strands 2a is equal to the total number of the second strands 2b. In the sparsely woven part 3c, the total number of the first strands 2a and the second strands 2b is smaller than that in the densely woven parts 3a and 3b. Therefore, the densely woven parts 3a and 3b have a higher strength and are less likely to be deformed than the sparsely woven part 3c.

In the densely woven parts 3a and 3b, for example, about several to several tens of the first strands 2a in total may be used. On the other hand, about several to several tens of the second strands 2b in total may be used.

Of the first strands 2a and the second strands 2b in the sparsely woven part 3c, the number of the first strands 2a having each the smaller diameter is less than the number of the second strands 2b having each the larger diameter.
In the sparsely woven part 3c, therefore, the number of the left-handed spiral protrusions formed of the second strands 2b is larger than the number of the right-handed spiral protrusions formed of the first strands 2a.
With this configuration, in the sparsely woven part 3c, the protruding shape of the left-handed spiral protrusion serving as the left-handed spiral deflector is further emphasized.

The difference in total number between the first strands 2a and the second strands 2b constituting the sparsely woven part 3c is preferably 2 to 12.

The stent of the embodiment may be manufactured as follows, for example. That is, a stent precursor is produced in the same manner as the manufacturing method for the stent of the example. Next, only a predetermined number of the first strands in a part to be the sparse part is cut and removed. As a result, the stent according to the embodiment of the present invention is obtained.

Alternatively, the stent of the embodiment may be manufactured as follows. That is, the stent weaving machine is used to weave the strands, thereby forming the stent in the same manner as the manufacturing method for the stent of the first embodiment. In this case, after one densely woven part (end part) is formed by weaving, the feeding of a predetermined number of the first strands is stopped. Next, the weaving is continued with the feeding of the first strands stopped. As a result, a sparsely woven part (central part) is formed. After that, the feeding of the first strands that has been stopped is restarted to continue the weaving. In this manner, the other densely woven part (end part) is woven. As a result, the stent according to the embodiment of the present invention is obtained.

The functions and effects of the stent of the embodiment will be described below.
The stent of the embodiment can exert the functions and effects (1) and (2) of the example described above. The stent of the embodiment can additionally exert the functions and effects (3) to (5).
(3) In the sparsely woven part of the stent of the embodiment, the protruding shape of the left-handed spiral protrusion serving as the left-handed spiral deflector is further emphasized.
   Therefore, in the case where the stent of the embodiment is indwelt at a lesion site, the bloodstream flows through the inner cavity of the densely woven part and is turned into a left-handed spiral flow. The resultant bloodstream then flows through the inner cavity of the sparsely woven part and is turned into a stronger left-handed spiral flow. As a result, the bloodstream is more stabilized.
(4) The densely woven parts, which have each more first strands and second strands and is less likely to be deformed, are each positioned at one end of the stent. Therefore, the stent indwelt at the lesion site is securely fixed at that position. As a result, the stent is hardly displaced.
(5) The sparsely woven part having a stronger deflecting effect is positioned at the central part of the stent. Therefore, the central part of the stent indwelt at an inlet of an aneurysm can easily cover the inlet of the aneurysm.

The stent indwelt in this manner makes it easy for the blood to flow along the stent. As a result, the bloodstream flowing into the aneurysm can effectively be prevented.

### (Other examples)

The following description refers to alternative, modified and/or additional features which may be applied selectively or in any technically feasible combination to each of the above described example and embodiment.

Of the first strands and the second strands in the stent of the present invention, the ratio between the (first) diameter of a thin strand and the (second) diameter of a thick strand is preferably 1:1.2 to 1:3.
In the case where the ratio between the diameter of the thin strand and the diameter of the thick strand is in the above range, a spiral deflector is formed by the thick strand more evidently protruding toward the virtual center (virtual central axis). This spiral deflector has a shape more suitable for adjusting the flow. Therefore, the bloodstream is even more stabilized.
In this case, the diameter of the thick strand is preferably 0.024 mm to 0.06 mm. The diameter of the thin strand, on the other hand, is preferably 0.02 mm to 0.05 mm.

In the stent of the present invention, the cross-sectional shape of the first strands and the second strands is not limited to circular, but may be oval or elliptic, for example. Therefore, the "diameter" corresponds to a maximum linear extension of the cross-sectional area of the respective one of the first and seconds strands, i.e., in analogy to the so-called Feret-diameter, to a longest distance between any two points on the contour of the cross-sectional area of the respective one of the first and seconds strands. Where the first and second strands have each a circular cross-sectional area, the "diameter" corresponds each to the diameter of the contour of the circular cross-sectional area of the first and second strands, respectively, and where the first and second strands have each a non-circular cross-sectional area, the "diameter" corresponds each to the diameter of a circle surrounding the contour of the non-circular cross-sectional area of the first and second strands, respectively.

In the stent of the present invention, the plurality of the first strands constituting the first strand group may be each formed of the same material. Alternatively, the plurality of the first strands may be formed of different materials, respectively.
The plurality of the second strands constituting the second strand group may be each formed of the same material. Alternatively, the plurality of the second strands may be formed of different materials, respectively.

In the stent of the present invention, the plurality of the first strands constituting the first strand group and/or the plurality of the second strands constituting the second strand group may include strands formed of radiopaque metal (e.g., platinum, gold, and tungsten).
In this case, the stent can easily be recognized in a contrast image. Therefore, the stent can easily be positioned when indwelt in a lesion site.

The outer surface of the stent of the present invention may be coated with a hydrophilic material.
The hydrophilic coating reduces the sliding resistance between the stent and a delivering catheter in which the stent is accommodated. As a result, the stent can smoothly be let out of the catheter and easily indwelt in a predetermined position.

The outer surface of the stent of the present invention may be coated with various chemicals. Examples of the chemical include sirolimus and paclitaxel which are used to prevent restenosis.

The stent of the present invention may be used for the purpose of being indwelt at an inlet of an aneurysm. Alternatively, the stent of the present invention may be used for the purpose of being indwelt in a tubular organ other than the vessel (e.g., urethra, bile duct, esophagus, intestine, and trachea).

A graft may be attached to the inner surface and/or outer surface of the stent of the present invention. In this case, the stent of the present invention can be used as a stent graft.
Examples of the material for the graft include at least one of polyester and PTFE.

### Reference Signs List

- 1, 3: stent
- 2A: first strand group
- 2a: first strand
- Da: first diameter of first strand
- 2B: second strand group
- 2b: second strand
- Db: second diameter of second strand
- L: virtual central axis
- 3a, 3b: densely woven part
- 3c: sparsely woven part

## Claims

1. A stent (3) comprising:
a first strand group (2A) constituted by a plurality of first strands (2a), and a second strand group (2B) constituted by a plurality of second strands (2b),
wherein the first stand group (2A) and the second strand group (2B) are woven together, and
wherein one of the first strand group (2A) and second strand group (2B) is wound in a right-handed spiral around a virtual central axis (L) that extends in a longitudinal direction of the stent (3), and the other one of the first strand group (2A) and second strand group (2B) is wound in a left-handed spiral around the virtual central axis (L),
the plurality of second strands (2b) constituting the second strand group (2B) are each larger in diameter than the plurality of first strands (2a) constituting the first strand group (2A), **characterized in that**
the number of the second strands (2b) is larger than the number of the first strands (2a),
a densely woven part (3a, 3b) is formed at each end of the stent (3) in a longitudinal direction thereof, which has a lamer total number of the first strands (2a) and second strands (2b),
a sparsely woven part (3c) is formed at a central part of the stent (3) in a longitudinal direction thereof, which has a smaller total number of the first strands (2a) and second strands (2b), and
in the sparsely woven part (3c) the number of the first strands (2a) is less than the number of the seconds strands (2b).

## Patentansprüche

1. Stent (3) mit:
einer aus einer Vielzahl von ersten Strängen (2a) gebildeten ersten Stranggruppe (2A) und
einer aus einer Vielzahl von zweiten Strängen (2b) gebildeten zweiten Stranggruppe (2B),
wobei die erste Stranggruppe (2A) und die zweite Stranggruppe (2B) miteinander verwoben sind,
wobei eine der ersten Stranggruppe (2A) und zweiten Stranggruppe (2B) in einer rechtsgängigen Wendel um eine in Längsrichtung des Stents (3) verlaufende virtuelle Mittelachse (L) und die andere der ersten Stranggruppe (2A) und zweiten Stranggruppe (2B) in einer linksgängigen Wendel um die virtuelle Mittelachse (L) gewickelt ist, und
wobei die Vielzahl von zweiten Strängen (2b), die die zweite Stranggruppe (2B) bilden, im Durchmesser jeweils größer sind als die Vielzahl von ersten Strängen (2a), die die erste Stranggruppe (2A) bilden, **dadurch gekennzeichnet, dass**
die Zahl der zweiten Stränge (2b) größer ist als die Zahl der ersten Stränge (2a),
an jedem Längenende des Stents (3) ein dicht gewebter Teil (3a, 3b) mit einer größerer Gesamtzahl an ersten Strängen (2a) und zweiten Strängen (2b) ausgebildet ist,
in einem Längenmittelteil des Stents (3) ein dünn gewebter Teil (3c) mit einer kleineren Gesamtzahl an ersten Strängen (2a) und zweiten Strängen (2b) ausgebildet ist, und
in dem dünn gewebten Teil (3c) die Zahl der ersten Stränge (2a) kleiner ist als die Zahl der zweiten Stränge (2b).

## Revendications

1. Stent (3), comprenant :
un premier groupe de brins (2A) constitué d'une pluralité de premiers brins (2a), et un second groupe de brins (2B) constitué d'une pluralité de seconds brins (2b), dans lequel le premier groupe de brins (2A) et le second groupe de brins (2B) sont tissés ensemble, et
dans lequel l'un du premier groupe de brins (2A) et du second groupe de brins (2B) est enroulé en une spirale dextrogyre autour d'un axe central virtuel (L) qui s'étend dans un sens longitudinal du stent (3), et l'autre du premier groupe de brins (2A) et du second groupe de brins (2B) est enroulé en une spirale lévogyre autour de l'axe central virtuel (L),
la pluralité de seconds brins (2b) constituant le second groupe de brins (2B) ont chacun un diamètre plus grand que la pluralité de premiers brins (2a) constituant le premier groupe de brins (2A), **caractérisé en ce que**
le nombre de seconds brins (2b) est plus grand que le nombre de premiers brins (2a),
une partie densément tissée (3a, 3b) est formée à chaque extrémité du stent (3) dans un sens longitudinal de celui-ci, laquelle a un plus grand nombre total de premiers brins (2a) et seconds brins (2b),
une partie peu densément tissée (3c) est formée au niveau d'une partie centrale du stent (3) dans un sens longitudinal de celui-ci, laquelle a un plus petit nombre total de premiers brins (2a) et seconds brins (2b), et
dans la partie peu densément tissée (3c) le nombre des premiers brins (2a) est inférieur au nombre de seconds brins (2b).
